Europäisches Patentamt

(19) European Patent Office

Office européen des brevets

(11) Publication number: **0 005 071**
**B2**

(12) # NEW EUROPEAN PATENT SPECIFICATION

(45) Date of publication of the new patent specification:
13.01.88

(21) Application number: 79300674.3

(22) Date of filling: 23.04.79

(51) Int. Cl.⁴: **A 61 B 10/00,** G 10 K 11/34,
G 01 S 7/52

---

(54) **Probe for electronic scanning type ultrasonic diagnostic apparatus.**

---

(30) Priority: 25.04.78 JP 48343/78

(43) Date of publication of application:
31.10.79 Bulletin 79/22

(45) Publication of the grant of the patent:
04.11.81 Bulletin 81/44

(45) Mention of the opposition decision:
13.01.88 Bulletin 88/2

(84) Designated Contracting States:
DE FR GB NL

(56) References cited:
DE-A-2 607 108
DE-A-2 718 772
DE-C-1 541 463
JP-A-49 091 390
US-A-3 718 898
US-A-3 936 791

Ultrasonics, January 1976, pages 29-33
Ultrasonics, July 1968, pages 153-159
IEEE Transactions on Sonics and Ultrasonics, Vol.
SU-19, October 1972, Nr. 4, pages 444-446

(73) Proprietor: Kabushiki Kaisha Toshiba, 72,
Horikawa- cho Saiwai- ku, Kawasaki- shi
Kanagawa- ken 210 (JP)

(72) Inventor: Takamizawa, Kinya, 873- 5, Kuden- cho
Totsuka- ku, Yokohama- shi Kanagawa- ken (JP)
Inventor: Uchiumi, Isao, 3155- 65, Mutsuura- cho
Kanazawa- ku, Yokohama- shi Kanagawa- ken (JP)

(74) Representative: Kirk, Geoffrey Thomas,
BATCHELLOR, KIRK & EYLES 2 Pear Tree Court
Farringdon Road, London EC1R 0DS (GB)

EP 0 005 071 B2

## Description

The present invention relates to a probe for electronic scanning type ultrasonic diagnostic apparatus according to the first part of claim 1. Such a probe is known from JP-A-49 91390.

More particularly, the invention is concerned with such apparatus which comprises a linear array of similar electro-acoustic transducers having electrical connections for transmitting and receiving ultrasonic waves, the transducers being arranged in a row on a supporting plate with equal intervals between consecutive transducers. Such a linear array and the means for energising the transducers are disclosed in figure 1 of US-A-3936791, for example.

Normally, the transducers are constituted by piezoelectric resonators which jointly direct pulses of ultrasound to that portion of the body to be examined, and subsequently detect the ultrasound pulse reflected at the boundaries between the organisms of the body. By varying the direction of the ultrasonic pulses directed into the body, information on the two-dimensional structure of the organisms is obtained and can be displayed on a CRT. The probe having the linear array of electro-acoustic transducers is normally separate from the electronic equipment of the diagnostic apparatus and is connected to that equipment by an electric cable, facilitating the movement of the probe over the body.

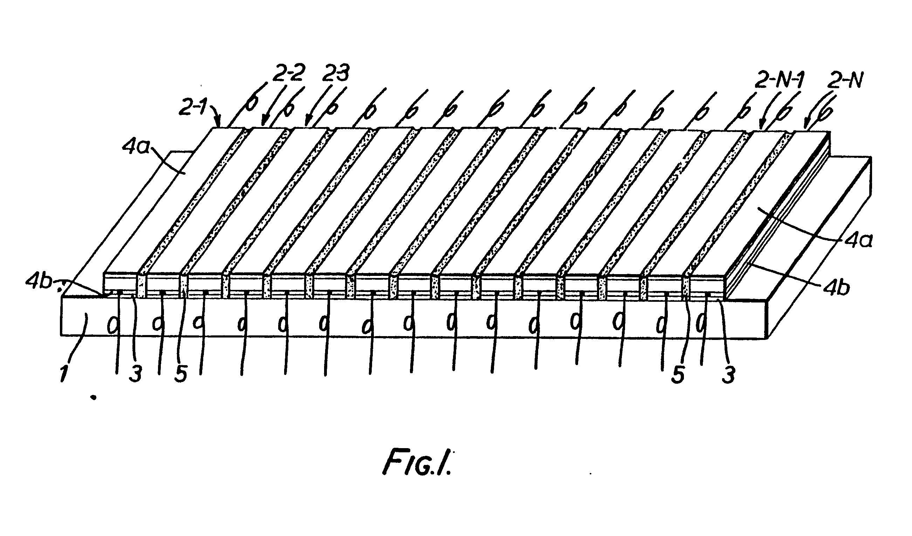

In order to vary the direction of transmission and reception of the ultrasound pulses, the transducers can be mechanically altered in direction. However, in the electronic scanning-type of ultrasonic apparatus to which this invention relates, varying the direction of transmission and reception is performed by energising the transducers sequentially, which is known as linear scanning and which is exemplfied in U.S. Patent No. 3789833, or by having progressive phase changes between the transducers so that the emitted wave front is angled to the probe by an angle which is dependent on the relative phases of the transducers. The latter type is known as sector scanning and is illustrated in Figure 1 of the above-mentioned U.S. patent specification.

Known probes for electronic scanning-type electronic diagnostic apparatus have suffered from the fault that a high quality image is not obtainable, due to the fact that the directivity of the ultrasound transmission from the transducers at or adjacent the ends of the row differs from that of the transducers intermediate the ends. The reason for this is that, although the central transducers are all flanked on each side by the neighbouring transducers, each end transducer is flanked by neighbouring transducers only on one side. The end transducers therefore have transmission and reception characteristics differing from the central transducers and in particular have asymmetrical directivity. For this reason the prior art devices provide undriven pseudo transducers on either side of the array of transducers.

An object of the present invention as claimed is to provide a probe for ultrasonic diagnostic apparatus in which all the transducers of the linear array have approximately the same directivity charecteristics and the transducers and pseudotransducers are protected against mechanical shocks.

The linear array may be provided with a known acoustic focussing lens, in order to focus the transmission in the direction normal to the length of the array; in that case, the acoustic lens preferably extends over all the transducers and also the pseudo-transducers and vibratory elements.

The invention will be more readily understood by way of example from the following description of probes for ultrasonic diagnostic apparatus, reference being made to the accompanying drawings, in which

Figure 1 is a perspective view showing the structure of a prior art probe;

Figure 2 is a similar view showing the structure of a prior art probe.

Figure 3 compares the directivity characteristics of the transducers at the ends of the row in the prior art probes with and without pseudo transducers.

Figure 4 is a perspective view illustrating a probe according to the invention.

Figures 5A and 5B are respectively a horizontal cross sectional view and a vertical cross sectional view of a still further modification; and

Figure 6 is a block diagram of the electronic equipment of a sector scanning type ultrasonic diagnostic apparatus, utilising a probe of the present invention.

The prior art probe shown in Figure 1 has a supporting plate 1, which is made of a material which absorbs ultrasound and a linear array of piezoelectric ultrasonic transducers 2-1 to 2-N secured on the supporting plate 1 by backing material 3. In order that the transducers may oscillate in the direction of their thickness each piezoelectric transducer has electrodes 4a and 4b on their upper and lower surfaces. The transducers 2-1 to 2-N are separated by spacers 5 of ultrasonic absorbing material and the upper exposed surfaces of the electrodes 4a are covered with a coating layer (not shown), for example epoxy resin, with protects the transducers and provides good and comfortable contact with the body to be examined.

In use, the upper electrodes 4a are earthed, while the electrodes 4b are individually supplied with high voltage pulses causing the transducers to resonate and emit ultrasound, or have electric signals corresponding to the reflected and received ultrasonic waves extracted therefrom. Although the spacers 5 and the supporting layer 1 are designed to minimise the mutual affect of the transducers on one another, in fact the oscillation of each transducer does affect the oscillation of neighbouring transducers, due to the supporting plate 1, backing material 3, spacers 5 and the coating layer; in other words, when each transducer oscillates, it interferes with its neighbouring transducers. Those transducers which are near to the centre of the linear array generally have similar ultrasonic directivities, since each is arranged with approximately the same configuration and environment on both sides. Each central transducer thus exhibits symmetrical directivity.

However, the transducers near the ends of the row, and particularly the end tranducers 2-1 and 2-N, while having similar transducers on one side, have no transducers on the other side. The ultrasonic directivity of each end transducer is thus different from that of the more central transducers and in fact each end transducer displays asymmetrical directivity which exerts a harmful influence on the directivity characteristics of the transmission and reception of the ultrasonic waves from and to the array as a whole. As a result, sectional image of high quality cannot be obtained when the prior art probe of Figure 1 is employed.

In sector scanning type apparatus, in which all transducers are simultaneously energised, the transmission of each transducer should be as far as possible non-directional. However, as above explained in the prior art probe the directivity characteristics of the end transducers differs from that of the central transducers and the adverse affect on the image, described above, is particularly noticeable in the case of such sector-scanning type equipment.

Even when a known acoustic focussing lens is employed on the transducers in order to sharpen the directivity characteristics in the plane normal to the length of the array deterioration of image quality is still apparent, because of the asymmetrical nature of the directivity characteristics of the transducers at or adjacent the ends of the array.

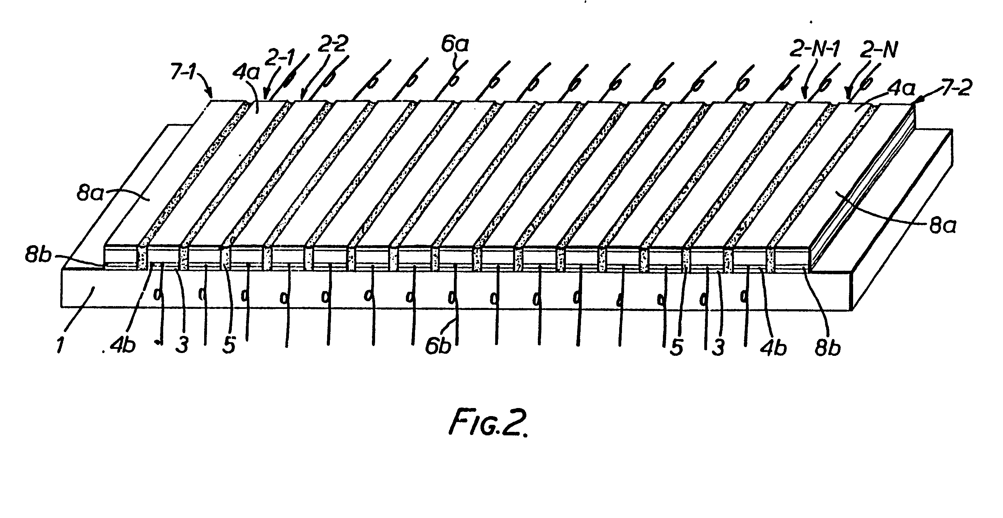

Turning now to the embodiment of the invention illustrated in Figure 2, the structure of the probe is generally similar to that of Figure 1 and corresponding parts in the two figures are given the same reference numerals. The upper electrodes 4a or the transducers are earthed through the leads 6a, while the lower electrodes 4b are individually connected to the pulse generating and receiving equipment through leads 6b.

The prior art probe of Figure 2 differs from that of Figure 1 in that a pseudo-transducer 7-1 or 7-2 is mounted adjacent the end transducers 2-1 or 2-N. Each of the pseudo-transducers 7-1 and 7-2 is made of the same vibratory material as the transducers 2, and has almost the same shape and dimensions as those of the transducers 2-1 to 2-N, while the distance between each pseudo-transducer and its adjacent transducer is approximately the same as the intervals between consecutive transducers 2. Each pseudo-transducer has on its upper and lower surfaces electrodes 8a and 8b which are similar to the electrodes 4a and 4b, and each pseudo-transducer is fixed to the supporting plate 1 by means of backing material 3 in the same manner as the transducers 2. A spacer is inserted between each pseudo-transducer 7 and its adjacent transducer 2, that spacer being similar to the spacers bete,een consecutive transducers 2.

In the probe of Figure 2, only the transducers 2-1 to 2-N are driven, no energisation being supplied for the electrodes 8b of the pseudo-transducers which in use vibrate in sympathy with the adjacent transducers 2. Since the pseudo-transducers 7-1 and 7-2 have approximately the same shape and dimensions as that of the transducers 2, the end transducers, for example 2-1, 2-2, 2-N-1 and 2-N, have the same directivity characteristics as the more central transducers. By providing the pseudo-transducers adjacent the ends of the row, all the transducers 2-1 to 2-N thus have symmetrical directivity characteristics.

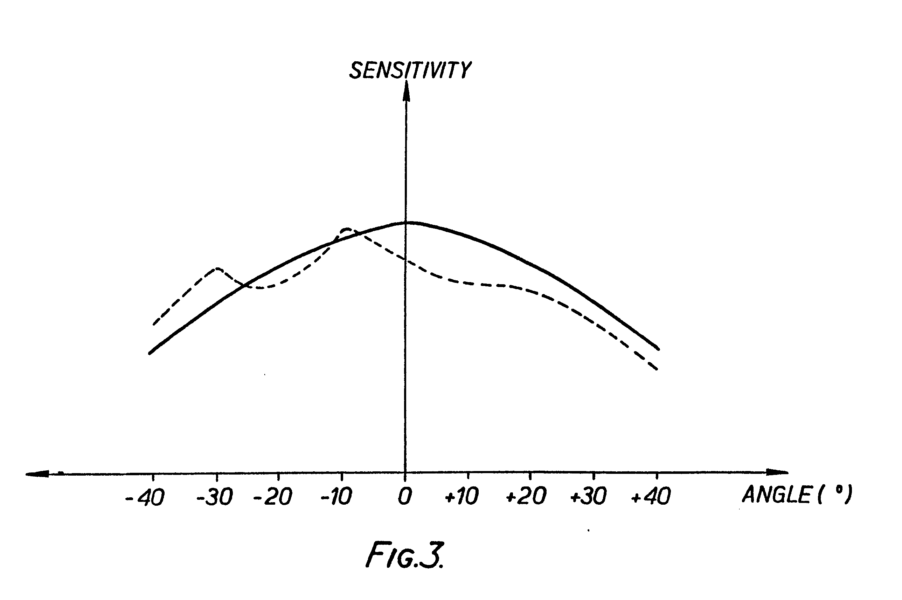

In Figure 3, the interrupted line shows the directivity characteristics of the transducers 2-1, of the prior art Figure 1, and the solid line shows the directivity characteristics of the transducer 2-1 of Figure 2. Also in Figure 3, the horizontal axis represents the angle and the vertical axis the sensitivity, the curves representing the variation in sensitivity with variation in the angle the direction of transmission or reception makes with the direction normal to the length of the probe. In both cases, the width of each of the transducers of Figure 1 and of the transducers and pseudo-transducers of Figure 2 is approximately 0.4 mm and the frequency of the ultrasonic waves is 2.4 MHz.

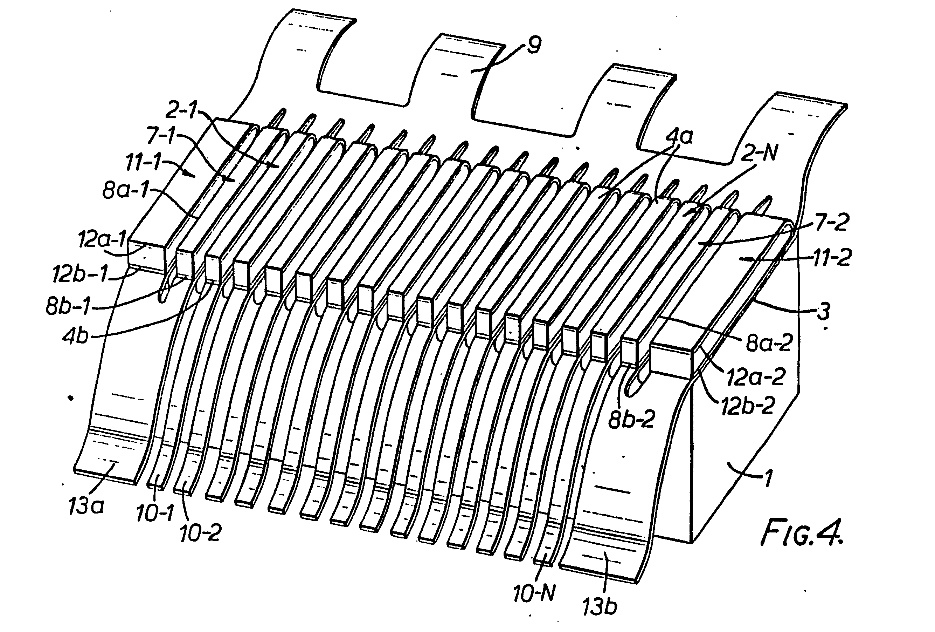

Figure 3 shows that the transducer 2-1 of Figure 2 has symmetrical directivity characteristics, whereas the corresponding characteristics of transducer 2-1 of Figure 1 is far from symmetrical. Consequently, a high quality sectional image is obtainable from the Figure 2 probe, when used in electronic scanning ultrasonic diagnostic appratus, especially of the sector-scanning type. Elements of vibratory material are provided additional to the pseudo-tranducers 7. Further, electrodes and conducting leads can be provided on both the pseudo-transducers and the additional vibratory elements. Such an embodiment of the invention is shown in Figure 4. As before, the array of transducers 2-1 to 2-N is mounted on the absorbing plate 1, which may be rubber -ferrite. The transducers 2 are made of piezo -electric ceramic and the width of each transducer is about 300 $\mu$, while the pitch is 370 $\mu$. The electrodes 4a in this instance are turned over the ends of the transducer to the lower surface, and are connected to a common earth lead 9. Electrodes 4b are connected to individual leads 10-1 to 10-N. Pseudo-transducers 7-1 and 7-2 are provided as before. In addition, further vibratory elements 11-1 and 11-2 are mounted on the plate 1 on the side of each pseudo-transducer 7 distant from the adjacent transducer 2-1 or 2-N. Pseudo-transducers 7-1 and 7-2 have the same shape, dimensions and characteristics as the transducers 2, but the width of each vibratory element 11-1 and 11-2 is wider than the pseudo-transducers, but are made of the same material as the pseudo-transducers 7 and the transducers 2. Upper electrodes 8a-1 and 8a-2 and lower electrodes 8b-1 and- 8b-2 are provided on the pseudo-transducers 7, while upper electrodes 12a-1 and 12a-2 and lower electrodes 12b-1 and 12b-2 are similarly provided on the upper and lower surfaces of the vibratory elements 11-1 and 11-2. Electrodes 8a-1 and 12a-1 are connected to the common earth lead 9, while electrodes 8b-1 and 12b-1 are connected to each other by a lead 13a; similarly electrodes 8b-2 and 12b-2 are connected to one another by lead 13b. The common lead 9, leads 10-1 to 10-N and leads 13a and 13b are made of copper leaf of 50 μ thickness. While the leads 9, 13a and 13b are earthed, leads 10-1 to 10-N are individually connected to pulse generators and limiters, as shown in Figure 7. High voltage pulses with different phases are applied to the respective transducers 2-1 to 2-N through leads 10-1 to 10-N, and as a result ultrasonic wave pulses are emitted from the linear array in the prescribed direction. Similarly, ultrasonic waves reflected from the body are received by the transducers 2-1 to 2-N and are converted to electrical signals in order to display the sectional image of the body.

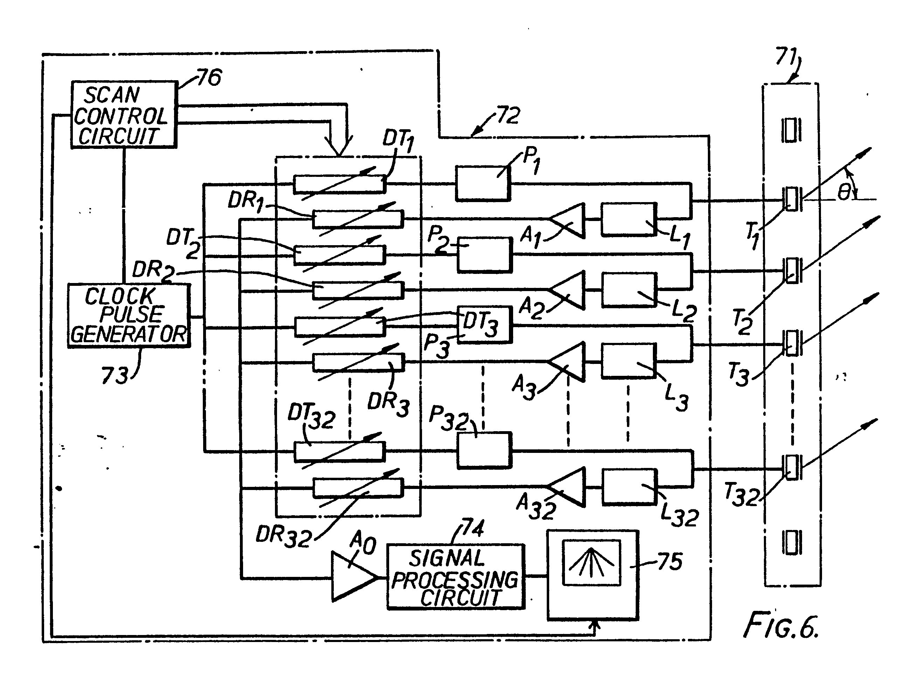

The vibratory elements 11-1 and 11-2 do not affect the directivity characteristics of the probe to any significant extent, but they protect the pseudo-transducers 7 and the transducers 2 against any mechanical shock to which the probe may be subject. In addition, when the probe has an overlying acoustic lens, as in Figures 6A and 6B, the vibratory elements 11 serve to support the ends of the lens.

In order to make the probe shown in Figure 4, a plate of vibratory material is taken and has applied to it, firstly, a sheet of copper having a pattern corresponding to the common lead 9 and, secondly, a sheet of copper leaf having a predetermined pattern corresponding to the leads 13a, 13b and 10-1 to 10-N. Next, the vibratory plate with the copper sheets thereon is fixed to the absorbent base plate 1 by means of the backing layers 3. The vibratory plate is cut into a number of pieces and separated electrically from one another, in order to form the independent transducers 2-1 to 2-N, the pseudo-transducers 7-1 and 7-2, and the vibratory elements 11-1 and 11-2. In this way, the various elements of the linear array are made simultaneously and with ease.

It is not necessary that the pseudo-transducers 7-1 and 7-2 should have the electrodes 8a-1, 8a-2, 8b-1 and 8b-2.

Although the directivity characteristics of the probe is improved as the number of pseudotransducers increases, in practice only one pseudo-transducer 7 at each side of the transducer array is sufficient.

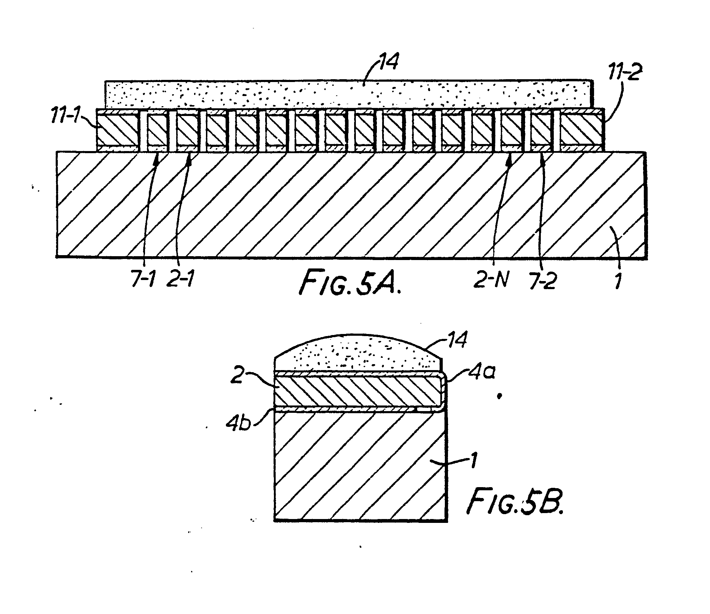

In the further embodiment shown in Figures 5A and 5B both pseudo-transducers 7-1 and 7-2 and vibratory elements 11-1 and 11-2 are again illustrated. In addition, the figures show an acoustic lens 14 which is secured over the transducers 2-1 to 2-N, the pseudo-transducers 7 and the vibratory elements 11. The lens 14 has a convex surface and is made from silicon rubber, and it concentrates the ultrasonic waves in the plane normal to the lenght of the row.

As will be observed, the acoustic lens 14 extends over not only the transducers 2 but also the pseudo-transducers 7 and the vibratory elements 11. Consequently, ultrasonic waves transmitted from the end tranducers 2-1 and 2-N, and ultrasonic waves received by those transducers, necessarily pass throgh the lens 14, and good directivity characteristics and concentration are obtained. Althogh a convex acoustic lens is shown, a concave acoustic lens may be alternatively used when the lens is made of a material in which the propagation velocity is faster than in the living body.

Figure 6 represents sector-scanning type ultrasonic diagnostic apparatus in which a probe is described above in relation to Figures 3 to 5 is employed. In this Figure the probe is shown at 71 and the console of the apparatus at 72. In the console there is a clock pulse generator 73 which generates standard pulses; adjustable delay circuits DT1 to DT32 connected to the generator 73 and outputting the pulses from the generator 73 with predetermined delays; pulse generators P1 to P32 which are driven by the outputs from the delay circuits DT1 to DT32 and which deliver high voltage pulse signals to electro-acoustic transducers T1 to T32 on the probe 71; limiters L1 to L32 for limiting signals received by the transducers T1 to T32 amplifiers A1 to A32 which amplify the signals from the limiters L; further adjustable delay circuits DR1 to DR32 which output the signals from amplifiers A1 to A32 with predetermined delays; an amplifier AO to the input of which the outputs from the delay circuits DR1 to DR32 are applied; a signal processing circuit 74 which processes the output signals from amplifier AO; a display 75 for displaying a sectional image of the examined body in accordance with the output signals from the circuit 74; and a scan control circuit 76 for controlling the delay times of the delay circuits DT1 to DT32 and DR1 to DR32 and for scanning the display 75.

The direction of the ultrasound pulses transmitted from the transducers T1 to T32 depends on the relative delays given by the delay circuits DT1 to DT32. When the difference between the delay time of the i-th delay circuit DTi and that of the i-l-th delay circuit DTi-l is

$$\frac{d}{c}\sin\theta,$$

the ultrasonic waves transmitted from the transducers T are approximately in the direction of the azimuth angle θ, where d represents the centre to centre distance between adjacent transducers T and c is the sound velocity in the medium. Further, in order to receive the ultrasonic waves reflected to the transducer from that direction, the delay circuits DR1 to DR32 have the same relative delays as the DT delay circuits.

The operation of the equipment is as follows. On the generation of the first pulse from the pulse generator 73, the control circuit 76 operates to set to zero the delay time of delay circuits DT32 and DR32, and to set a delay time of

$$\frac{d}{c}\sin\theta 1$$

to the delay circuits DT31 and DR31, a delay of

$$\frac{2d}{c}\sin\theta 1$$

to the delay circuits DT30 and DR30 and correspondingly for the remaining delay circuits.

The first clock pulse from generator 73 is supplied to the pulse generators P1 to P32 through the delay circuits DT1 to DT32 and, as a result, high voltage pulses generated from the pulse generators P are supplied to the corresponding transducers T1 to T32 respectively, so that an ultrasonic beam is transmitted in the direction of θ1. The ultrasound waves reflected from the body are received by the transducers T1 to T32 and converted to electric signals. Those signals pass through the limiters L1 to L3, the amplifiers A1 to A32 and the delay circuits DR1 to DR32. The mixed signal from delay circuits DR then represents the intensity of the ultrasound waves reflected in the direction of θ1. The mixed signal from the delay circuits DR is again amplified by the amplifier AO and processed by the processing circuit 74, before being supplied to the display 75.

The second clock pulse from generator 73 controls the delay circuits DT and DR so that the ultrasound beam from the transducers T is transmitted in the direction of θ2. Consequently, electric signals corresponding to the reflected ultrasound waves in the direction of θ2 is supplied to the display 75 as before. In like manner, the delay times are changed with every clock pulse generated from the generator 73, so that the angle of scanning is altered progressively and a sectional image of the body is displayed on display 75.

The system described in relation to Figure 6 is of the sector scanning type. However, it will be apparent that the probe can be used in linear scanning type ultrasonic diagnostic apparatus.

## Claims

1. A probe for electronic scanning type ultrasonic diagnostic apparatus comprising a linear array of similar electro-acoustic transducers (2-1 to 2-N) having electrical connections for transmitting and receiving ultrasonic waves, the transducers being arranged in a row on a supporting plate (1) with equal intervals between consecutive transducers, and at least one undriven pseudo-transducer (7-1, 7-2), which has approximately the same shape and dimensions as the transducers and is made of the same vibratory material as the transducers, is arranged on the supporting plate (1) adjacent each end transducer (2-1, 2-N) of the row with the same interval between the pseudo-transducer and the end transducer as the interval between consecutive transducers, whereby the ultrasonic directivity characteristics of the end transducers (2-1, 2-N) are similar to those of the other transducers, characterised in that one or more vibratory elements (11-1, 11-2) are provided adjacent to each of the pseudo-transducers (7-1, 7-2), on the side thereof distant from the transducers (2), those elements (11-1, 11-2) being wider than the pseudo-transducers (7-1, 7-2).

2. A probe for electronic scanning type ultrasonic diagnostic apparatus as claimed in claim 1, wherein each of the pseudo-transducers (7-1, 7-2) has a pair of electrodes (8).

3. A probe for electronic scanning type ultrasonic diagnostic apparatus according to claim 1 wherein each of the vibratory elements (11-1, 11-2) has a pair of electrodes.

4. A probe for electronic scanning type ultra sonic diagnostic apparatus according to claim 1,2 or claim 3, wherein an acoustic lens (14) for concentrating and receiving ultrasonic waves is superimposed over the transducers (2), the pseudo-transducers (7) and the vibratory elements (11).

## Patentansprüche

1. Sonde für ein elektronisch abtastendes Ultraschall-Diagnosegerät, die eine geradlinige Anordnung gleichartiger elektroakustischer Umformer (2-1 bis 2-N) umfaßt, welche elektrische Verbindungen zur Übertragung und zum Empfang von Ultraschallwellen aufweist, wobei die Umformer in einer Reihe auf einer Trägerplatte (1) mit gleichen Abständen zwischen aufeinanderfolgenden Umformern angeordnet sind und wenigstens ein nichtgetriebener Pseudo-Umformer (7-1, 7-2) jedem End-Umformer (2-1, 2-N) der Reihe benachbart auf der Trägerplatte (1) angeordnet ist, der ungefähr gleiche Form und Abmessungen aufweist wie die Umformer und aus demselben schwingungsfähigen Werkstoff wie die Umformer hergestellt ist, wobei der gleiche Abstand zwischen dem Pseudo-Umformer und dem End-Umformer vorgesehen ist wie zwischen aufeinanderfolgenden Umformern, wobei die Ultraschall-Richtcharakteristik der End-Umformer (2-1, 2-N) der der anderen Umformer entspricht, dadurch gekennzeichnet, daß ein oder mehrere schwingfähige Elemente (11-1, 11-2) jedem der Pseudo-Umformer (7-1, 7-2) benachbart an deren von den Umformern (2) abgewandter Seite vorgesehen sind und daß diese Elemente (11-1, 11-2) breiter als die Pseudo-Umformer (7-1, 7-2) sind.

2. Sonde nach Anspruch 1, worin jeder der Pseudo-Umformer (7-1, 7-2) ein Paar Elektroden (8) aufweist.

3. Sonde nach Anspruch 1, worin jedes der schwingungsfähigen Elemente (11-1, 11-2) ein Paar Elektroden aufweist.

4. Sonde nach Anspruch 1, 2 oder 3, bei welcher über den Umformern (2), den Pseudo-Umformern (7) und den schwingfähigen Elementen (11) eine akustische Linse (14) zum Konzentrieren und Empfangen von Ultraschallwellen angeordnet ist.

**Revendications**

1. Sonde pour un appareil de diagnostic électronique du type à analyse électronique qui comprend un arrangement linéaire de transducteurs électroacoustiques similaires (2-1 à 2-N) ayant des connexions électriques pour transmettre et recevoir des ondes ultrasoniques, ces transducteurs étant disposés en une rangée sur une plaque de support (1) en ménageant des intervalles égaux entre les transducteurs consécutifs et, au moins, un pseudo-transducteur inactif (7-1, 7-2) qui a approximativement la même forme et les mêmes dimensions que les transducteurs et est fait de la même matière vibrante que ces derniers, est monté sur la plaque de support (1) près de chaque transducteurs d'extrémité (2-1, 2-N) de la rangée avec le même intervalle entre le pseudo-transducteur et le transducteur d'extrémité qu'entre les transducteurs consécutifs, ce qui fait que les caractéristiques de directivité des transducteurs d'extrémité (2-1, 2-N) sont les mêmes que celles des autres transducteurs, caractérisée en ce qu'un ou plusieurs éléments vibrants (11-1, 11-2) sont prévus près de chaque pseudo-transducteur (7-1, 7-2) du côté de celui-ci qui est éloigné des transducteurs (2), ces éléments (11-1, 11-2) étant plus larges que les pseudo-transducteurs (7-1, 7-2).

2. Sonde pour un appareil de diagnostic électronique du type à analyse électronique selon la revendication 1, caractérisée en ce que chaque pseudo-transducteur (7-1, 7-2) possède deux électrodes (8).

3. Sonde pour un appareil de diagnostic ultrasonique du type à analyse électronique selon la revendication 1, caractérisée en ce que chacun des éléments vibrants (11-1, 11-2) a une paire d'électrodes.

4. Sonde pour un appareil de diagnostic ultrasonique du type à analyse électronique selon les revendications 1, 2 ou 3 caractérisée en ce qu'une lentille acoustique (14) pour concentrer et recevoir les ondes ultrasoniques est superposée sur les transducteurs (2), les pseudo-transducteurs (7) et les éléments vibrants (11).

FIG.1.

FIG.2.

FIG.3.

FIG.4.

FIG.5A.

11-1
11-2
14
7-1  2-1  2-N  7-2  1

FIG.5B.

14
2
4a
4b
1

Fig.6.